# EUROPEAN PATENT APPLICATION

(11) **EP 3 406 200 A1**
(43) Date of publication of application: **28.11.2018**
(21) Application number: 17770317.0
(22) Date of filing: 23.03.2017
(51) Int. Cl.: A61B 10/02

(54) **TISSUE SAMPLING DEVICE**

(30) Priority: 23.03.2016 JP 2016058142
(71) Applicant: Terumo Kabushiki Kaisha, Tokyo 151-0072 (JP)
(72) Inventor: YORI, Kouichirou, Ashigarakami-gun Kanagawa 259-0151 (JP)
(74) Representative: Prüfer & Partner mbB Patentanwälte · Rechtsanwälte
(86) International application number: PCT/JP2017/011624
(87) International publication number: WO 2017/164277

(57) **Abstract**

To provide a device that can quantitatively collect tissue by a simple operation.

The tissue collection device includes a sheath member having a first threaded portion on an inner face thereof, and a puncture tube fitted for back and forth movement in the sheath member and having a second threaded portion provided on an outer face thereof for threaded engaging with the first threaded portion. The sheath member and/or the puncture tube has a blade at a distal thereof.

## Description

### Technical Field

The present invention relates to a device capable of performing tissue collection quantitatively.

### Background Art

Regenerative medical products are obtained by performing a process such as cultivation for human cells using a human tissue or the like as a raw material. In order to produce regenerative medical products, it is necessary to secure an amount of tissue necessary for the production, and therefore, the human body is incised by a scalpel or the like to collect the tissue. However, such a method as just described is high in invasiveness and has a large impact on the human body.

Meanwhile, as low invasive means, a tissue collection needle for biopsy or the like is available. However, this is for collecting a very small amount of tissue for inspection to the last, and in order to secure an amount of tissue necessary for a regenerative medical product, it is necessary to use such a needle as descried above by a plural number of times for the human body.

For example, Patent Document 1 discloses a cell tissue collector that includes a tubular body having a distal formed in a shape of a needle of a syringe, and a brush bar having a distal formed in a shape of a needle and having brush hair provided in a projecting manner in the proximity of the distal thereof. According to this cell tissue collector, the brush bar is disposed for fitting into the tubular body such that the brush hair and the distal needle are moved into and out of the tubular body to collect cells from a wide range in an internal organ. However, such a problem that the cell tissue collector is high in invasiveness or the like is concerned.

Patent Document 2 discloses a biopsy device that includes a tube shaped sheath and a core shaft configuring a needle main body having a spiral groove at a distal portion thereof. According to this device, the core shaft is moved into and out of the sheath to catch tissue into the spiral groove. However, since the core shaft occupies the volume in the sheath, such a problem that the collection amount is small or the like is concerned.

Patent Document 3 discloses a puncture needle that includes a sheath member, a needle tube, and a needle member having a rotary blade and in which graduations are provided on a needle member operation unit and a needle tube operation unit. According to the puncture needle, it is tried to adjust the length over which the needle member projects with the graduations. However, since such adjustment relies upon the manipulation of the operator, such a problem that tissue cannot be collected quantitatively or the like is concerned.

### Prior Art Document

### Patent Documents

Patent Document 1: Japanese Patent Laid-open No. 2000-60859
Patent Document 2: Japanese Patent Laid-open No. 2012-235878
Patent Document 3: Japanese Patent Laid-open No. 2015-85141

### Summary of Invention

### Technical Problems

Under such circumstances, the inventor of the present invention has faced, in development of a device that performs tissue collection efficiently, such problems that a conventional biopsy device that collects tissue through back and forth movement of a needle member cannot secure a sufficient amount of tissue by a single operation, that there is a difference in collection amount depending upon the manipulation of the operator, and so forth. Accordingly, the object of the present invention resides in provision of a device that solves such problems as described above and can collect tissue quantitatively by a simple operation.

### Technical Solution

The inventor of the present invention has paid attention, while continuing to conduct intensive research in order to solve the aforesaid subject, to that, in order to prevent occurrence of a difference in collection amount by the manipulation, a device with which any person can quantitatively collect tissue by a simple operation must be developed. Then, as a result when the research was continuously conducted, the inventor of the present invention has found that tissue can be collected quantitatively by controlling forward and backward movement of a tissue collection device by a threaded engagement mechanism, and has completed the present invention.

In particular, the present invention relates to the followings.
[1] A tissue collection device, including a sheath member having a first threaded portion on an inner face thereof, and a puncture tube fitted for back and forth movement in the sheath member and having a second threaded portion provided on an outer face thereof for threaded engaging with the first threaded portion,
   in which the sheath member and/or the puncture tube has a blade at a distal thereof.
[2] The tissue collection device according to aforesaid [1], in which the puncture tube has a spiral blade provided in a projecting manner on an inner face of the puncture tube.
[3] The tissue collection device according to aforesaid [1] or [2], in which the sheath member has graduations on an outer face thereof.
[4] The tissue collection device according to any one of aforesaid [1] to [3], in which the puncture tube includes an outer side tubular body and an inner side tubular body fitted for back and forth movement in the outer side tubular body.
[5] The tissue collection device according to aforesaid [4], in which the inner side tubular body has a groove extending in an axial direction thereof.
[6] The tissue collection device according to any one of aforesaid [1] to [5], further including:
   a vessel for accommodating the puncture tube, the vessel having a third threaded portion for threaded engaging with the second threaded portion.
[7] The tissue collection device according to any one of aforesaid [1] to [6], in which at least part is configured from an optically transparent material.

### Advantageous Effects

With the present invention, a device can be provided which can quantitatively collect tissue by a simple operation. Further, by using the device of the present invention, tissue can be quantitatively collected by a single time operation without depending upon the manipulation of the operator, and therefore, low invasiveness can be achieved.

### Brief Description of Drawings

[FIG. 1]

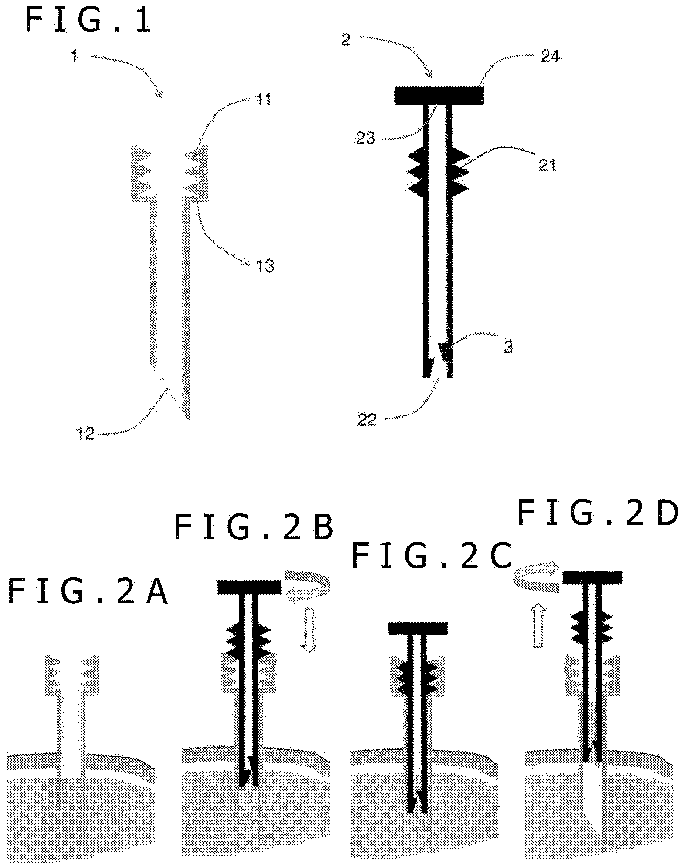

   FIG. 1 is a sectional view schematically depicting a device according to a first embodiment of the present invention.
[FIG. 2]
   FIGS. 2A to 2D are sectional views schematically depicting an example of use of the device according to the first embodiment of the present invention.
[FIG. 3]

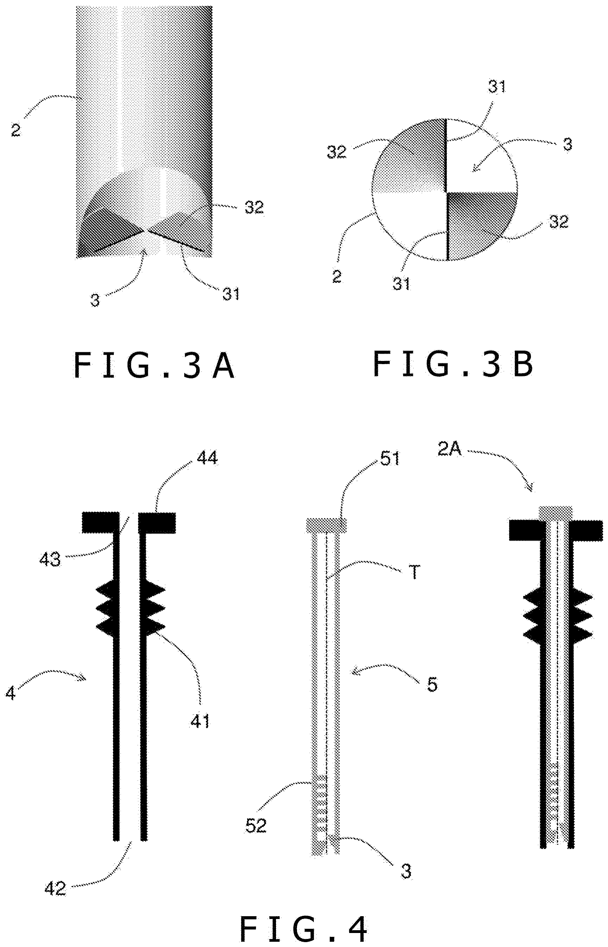

   FIGS. 3A and 3B are sectional views schematically depicting a spiral blade 3 of the present invention.
[FIG. 4]
   FIG. 4 is a sectional view schematically depicting a device according to a second embodiment of the present invention.
[FIG. 5]

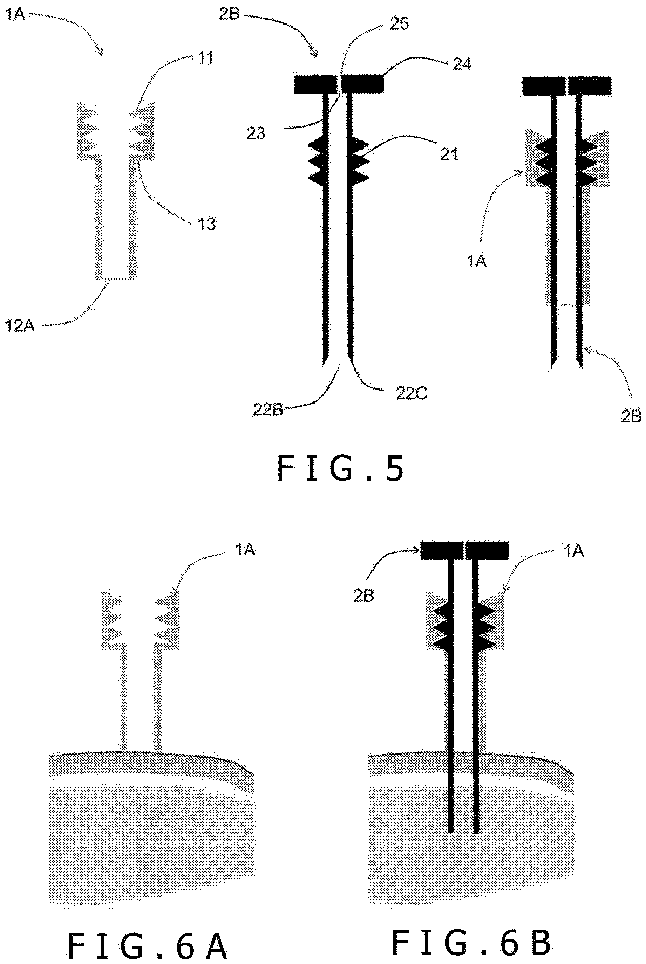

   FIG. 5 is a sectional view schematically depicting a device according to a third embodiment of the present invention.
[FIG. 6]
   FIGS. 6A and 6B are sectional views schematically depicting an example of use of the device according to the third embodiment of the present invention.
[FIG. 7]

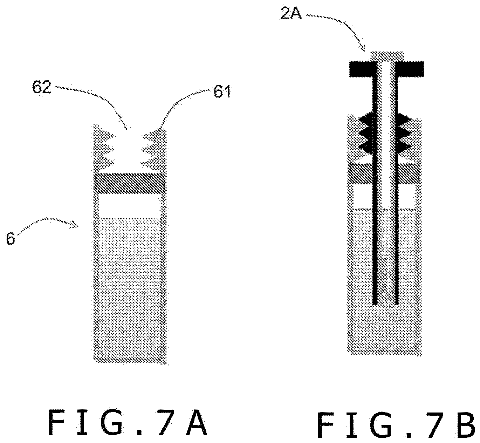

   FIGS. 7A and 7B are sectional views schematically depicting a vessel of the present invention.

### Modes for Carrying Out the Invention

In the following, the present invention is described in detail on the basis of referred embodiments with reference to the drawings. Note that the size of each member in the figures is suitably emphasized for description and does not indicate an actual ratio or magnitude. Further, in the following description, the term proximal portion signifies an end portion of the device on the operator side, and the term distal portion signifies an end portion of the device on the tissue side.

### [First Embodiment]

First, a device according to a first embodiment of the present invention is described. FIG. 1 is a sectional view schematically depicting the device according to the first embodiment of the present invention. The device includes a sheath member 1 and a puncture tube 2, and the puncture tube 2 can be fitted for back and forth movement in the sheath member 1. Both of the sheath member 1 and the puncture tube 2 are have a form of a tubular body. The sheath member 1 has a first threaded portion 11 provided in a projecting manner on an inner face on the proximal side thereof, and a distal portion 12 of the sheath member 1 is formed as a blade of a pointed shape that is inclined with respect to a plane perpendicular to a long axis of the sheath member 1 and can pierce the tissue. The puncture tube 2 has a second threaded portion 21 provided in a projecting manner on an outer face on the proximal side thereof for threaded engaging with the first threaded portion 11. The length of the first threaded portion 11 is substantially equal to the length of the second threaded portion 21, and the length of the distal side of the sheath member 1 with respect to the first threaded portion 11 is substantially equal to the length of the distal side of the puncture tube 2 with respect to the second threaded portion 21.

At a distal portion 22 of the puncture tube 2, a spiral blade 3 is provided in a projecting manner on the inner face, and an operation unit 24 is provided at a proximal portion 23 of the puncture tube 2. The outer diameter of the proximal side of the sheath member 1 on which the first threaded portion 11 is provided in a projecting manner is greater than the outer diameter of the sheath member 1 on the distal side with respect to the first threaded portion 11, and by this offset between the outer diameters, a stopper 13 is formed. Further, the inner diameter of the distal side of the sheath member 1 with respect to the first threaded portion 11 is designed such that it is smaller than the outer diameter of the second threaded portion 21 of the puncture tube 2 such that the second threaded portion 21 is not engaged with the distal side farther than the first threaded portion 11 of the sheath member 1.

FIGS. 2A to 2D are sectional views schematically depicting an example of use of the device according to the first embodiment of the present invention. In the case where the device of the present invention is to be used, the distal portion 12 of the sheath member 1 puncture an application region to perform incision and cutting out of the disuse and takes the tissue into the inner side of the sheath member 1 (FIG. 2A). Since the sheath member 1 has the stopper 13, such a problem that the sheath member 1 inadvertently enters the application region or a like problem is less likely to occur. Here, the sheath member 1 may have graduations on an outer face thereof such that the puncture depth of the sheath member 1 at the application region can be confirmed.

Then, the puncture tube 2 is inserted into the sheath member 1 until the second threaded portion 21 of the puncture tube 2 and the first threaded portion 11 of the sheath member 1 are threaded engaged with each other and the operation unit 24 is operated to rotate the puncture tube 2 (FIG. 2B). While the puncture tube 2 rotates, it is threaded engaged in a direction toward the distal of the sheath member 1 (in a downward arrow mark direction) and takes the tissue in the sheath member 1 into the inner side of the puncture tube 2 while the spiral blade 3 of the puncture tube 2 shreds the tissue in the sheath member 1. When the puncture tube 2 is to be rotated, preferably it is rotated in a state in which the sheath member 1 is fixed.

Since the inner diameter of the distal side of the sheath member 1 is smaller than the outer diameter of the second threaded portion 21 of the puncture tube 2, the threaded engagement of the puncture tube 2 is stopped in the middle (FIG. 2C). In particular, even if the operation unit 24 is operated further, the puncture tube 2 does not rotate and does not advance any more. When the tissue is to be taken out, either the sheath member 1 is pulled out in a direction of an upward arrow mark together with the puncture tube 2 or the puncture tube 2 is rotated in the opposite direction to pull out the puncture tube 2 in the direction of the upward arrow mark (FIG. 2D). When the puncture tube 2 is to be rotated so as to be pulled out, the sheath member 1 and the puncture tube 2 may be rotated integrally in advance such that the tissue in the puncture tube 2 is cut away with certainty from the application region by the spiral blade 3. Here, the sheath member 1 and the puncture tube 2 may be structured such that, according to the threaded engagement between them, if the puncture tube 2 is threaded engaged to a fixed position in the sheath member 1, then the puncture tube 2 begins to idly rotate and does not advance any more in the axial direction. In this case, as the puncture tube 2 idly rotates in the sheath member 1, it can cut away the tissue, and the tissue can be collected in lower invasiveness than that in an alternative case in which the puncture tube 2 is rotated together with the sheath member 1 to cut away the tissue.

As described above, since the device of the present invention restricts the back and forth movement of the puncture tube 2 by threaded engagement, such a problem that the puncture tube 2 inadvertently enters the application region or a like problem is less likely to occur. Further, since the distance of the back and forth movement of the puncture tube 2 depends upon the distance of rotation of the operation unit 24, for example, the collection amount of tissue can be adjusted quantitatively by the number of rotations of the operation unit 24. Further, since the stopper 13 is formed on the outer face of the sheath member 1 of the present invention, when the sheath member 1 punctures an application region, such a problem that the sheath member 1 inadvertently enters the application region or a like problem is less likely to occur.

Since the puncture tube 2 of the present invention is a tubular body, a sufficient amount of tissue can be taken into the inner side of the puncture tube 2. Further, since the spiral blade 3 is provided in a projecting manner on the inner face of the puncture tube 2, the puncture tube 2 advances while rotating in the application region and can shred the tissue. Further, the spiral blade 3 achieves a function of a lid at the distal portion 22 of the puncture tube 2, and when the sheath member 1 and/or the puncture tube 2 are to be pulled out in a state in which tissue is taken in the puncture tube 2, the spiral blade 3 can prevent the tissue from falling from the distal portion 22 of the puncture tube 2.

FIGS. 3A and 3B are partial sectional views schematically depicting the spiral blade 3 of the present invention. As depicted in FIG. 3A, the spiral blade 3 includes two plate-shaped members 32 each having a blade 31 at a distal thereof and inclined with respect to a plane perpendicular to the long axis of the puncture tube 2. The blade 31 is disposed such that it is inclined with respect to the plane perpendicular to the long axis of the puncture tube 2 and can cut obliquely into the surface of the application region to shred the tissue. The spiral blade 3 is provided in a projecting manner on an inner face of a distal portion of the puncture tube 2, and the length of the spiral blade 3 in the axial direction is smaller than the length of the puncture tube 2 in the axial direction. In particular, the spiral blade 3 is not provided on the proximal side with respect to the distal portion of the puncture tube 2 and does not make an obstacle when the tissue taken in the puncture tube 2 is to be taken out. Further, the spiral blade 3 is fixed to the inner face of the puncture tube 2 and a support does not occupy the volume of the puncture tube 2 like an auger screw, an amount of tissue greater as much can be taken in.

FIG. 3B is a view of the puncture tube 2 on which the spiral blade 3 is provided in a projecting manner as viewed from the axial direction. The plate-shaped members 32 have a sectoral shape of an angle of 90 degrees as viewed from the axial direction, and the two plate-shaped members 32 are provided in a projecting manner at positions opposing to each other on the inner face of the puncture tube 2. In other words, the two plate-shaped members 32 are disposed such that they draw a double spiral in the puncture tube 2 and can shred the tissue more finely. Further, the two blades 31 of the two plate-shaped members 32 are disposed such that they cross a diameter of the puncture tube 2, and when the puncture tube 2 is to be inserted into an application region while being rotated, the spiral blade 3 is likely to cut into the tissue. Furthermore, by the two plate-shaped members 32 opposing to each other, the tissue taken in the puncture tube 2 can be held with a higher degree of certainty.

In this manner, the device according to the first embodiment of the present invention can collect tissue quantitatively by a single time operation without depending upon the manipulation of the operator, and therefore, low invasiveness can be achieved.

Note that the sheath member 1 is not limited to that of the aforesaid embodiment but can assume various modifications. For example, the stopper 13 of the sheath member 1 is not limited to such a stopper that utilizes an aforesaid offset as described above, and, for example, a flange portion (not detected) may be provided which can be fixed to an outer face of the sheath member 1 but is slidably movable in the axial direction of the sheath member 1. By this, quantitative tissue collection can be achieved by slidably moving the flange portion with reference to such graduations on the outer face of the sheath member 1 as described above.

Further, the spiral pitch of the spiral blade 3 may be made equal to the thread pitch between the puncture tube 2 and the sheath member 1. Consequently, it is possible to minimize destruction of the tissue by the spiral blade 3 to advance the spiral blade 3 smoothly into the tissue. The angle of the member 32 configuring the spiral blade 3 as viewed from the axial direction is not limited to 90 degrees and can be set freely within the range of 1 to 360 degrees or exceeding 360 degrees. In particular, the spiral blade 3 may be a blade in the form of a string, a sectoral blade or a spiral blade of 360 degrees or more. Also the number of such members 32 is not limited to two but can be set freely, for example, to one to ten or more. Furthermore, the projection height of the spiral blade 3 in the center direction may be set greater than the radius of the puncture tube 2 to increase the cutting away effect of the tissue.

### [Second Embodiment]

Now, a device according to a second embodiment of the present invention is described. FIG. 4 is a sectional view schematically depicting the device according to the second embodiment of the present invention. Note that, in the following direction, like portions to those of the device according to the first embodiment are denoted by like reference characters and description of them is omitted.

In the present embodiment, a puncture tube 2A includes an inner side tubular body 5 (centrally in FIG. 4) and an outer side tubular body 4 (left side in FIG. 4). The inner side tubular body 5 is fitted for back and forth movement in the outer side tubular body 4 (right side in FIG. 4). The outer side tubular body 4 has an opening 43 on the proximal side thereof such that the inner side tubular body 5 can be fitted into the outer side tubular body 4 from the proximal side. The inner side tubular body 5 has a spiral blade 3 provided in a projecting manner on an inner face thereof, and the outer side tubular body 4 has a threaded portion 41 provided in a projecting manner on an outer face thereof. In the present embodiment, the first threaded portion 11 (not depicted) of the sheath member 1 (not depicted) is threaded engaged with the threaded portion 41 of the outer side tubular body 4 and tissue is taken into the inside of the inner side tubular body 5.

A grasping unit 51 is provided on the proximal side of the inner side tubular body 5, and the outer diameter of the grasping unit 51 is greater than the inner diameter of the outer side tubular body 4. Although an operator can grip the grasping unit 51 to slidably move the inner side tubular body 5 in the outer side tubular body 4, the advancement of the grasping unit 51 is stopped on the proximal side of the outer side tubular body 4. The grasping unit 51 can be removably fixed to an operation unit 44 of the outer side tubular body 4, and the outer side tubular body 4 and the inner side tubular body 5 can be rotated integrally.

A reduced thickness portion T extending in the axial direction is provided on the inner side tubular body 5. When tissue is to be taken out, by pulling out the inner side tubular body 5 from the outer side tubular body 4 and putting a bar-like member or air into the inner side tubular body 5 from the proximal side or by like means to push out the tissue to the distal side or dividing the inner side tubular body 5 longitudinally along the reduced thickness portion T, the tissue can be taken out readily. Further, the inner side tubular body 5 is configured from an optically transparent material and has graduations 52 on an outer face thereof. Accordingly, the operator can confirm the amount of the tissue taken in the inner side tubular body 5 from the outside of the inner side tubular body 5. Then, in the case where the collection amount is small, the tissue can be taken in by the insufficient amount by returning the inner side tubular body 5 into the outer side tubular body 4 and rotating the puncture tube 2A in the sheath member 1 again.

Since the device according to the second embodiment of the present invention can collect tissue quantitatively by a single time operation without depending upon the manipulation of the operator, low invasiveness can be achieved.

Note that the puncture tube 2A is not restricted to that of the aforesaid embodiments but can assume various modifications. For example, both the inner side tubular body 5 and the outer side tubular body 4 may be configured from an optically transparent material such that the collection amount of tissue can be confirmed only by taking out the puncture tube 2A from the sheath member 1. Furthermore, also the sheath member 1 may be configured from an optically transparent material such that the collection amount of tissue can be confirmed without taking out the puncture tube 2A from the sheath member 1.

### [Third Embodiment]

Now, a device according to a third embodiment of the present invention is described. FIG. 5 is a sectional view schematically depicting the device according to the third embodiment of the present invention. Note that, in the following direction, like components to those of the devices according to the first and second embodiments are denoted by like reference characters and description of them is omitted.

In the present embodiment, the length of a sheath member 1A (left side in FIG. 5) on the distal side with respect to a first threaded portion 11 is smaller than the length of a puncture tube 2B (centrally in FIG. 5) on the distal side with respect to a second threaded portion 21. A distal portion 12A of the sheath member 1A extends in parallel to a plane perpendicular to the long axis of the sheath member 1A. The distal portion 12A of the sheath member 1A is designed such that it has no blade provided thereon and, even if the distal portion 12A of the sheath member 1A is pressed against an application region, it does not pierce the application region. A distal portion 22B of the puncture tube 2B extends in parallel to a plane perpendicular to the long axis of the puncture tube 2B. In the present embodiment, the inner face of the puncture tube 2B has no spiral blade provided thereon, and instead, a blade 22C is provided at a lower end of the puncture tube 2B, namely, at the distal portion 22B. Accordingly, the distal portion 22B of the puncture tube 2B can puncture an application region to perform incision and cutting out of tissue and then take in the tissue into the inner side of the puncture tube 2B. An opening 25 is provided at a proximal portion 23 of the puncture tube 2B. If the puncture tube 2B is fitted into the sheath member 1A, then the distal side of the puncture tube 2B is exposed from the distal portion 12A of the sheath member 1A (right side in FIG. 5).

FIGS. 6A and 6B are sectional views depicting an example of use of the device according to the third embodiment of the present invention. In the case where the device of the present embodiment is to be used, the distal portion 12A of the sheath member 1A is placed on the surface of an application region (FIG. 6A). Since no blade is provided on the distal portion 12A, the sheath member 1A does not pierce the application region. Then, the puncture tube 2B is inserted into the sheath member 1A such that the second threaded portion 21 of the puncture tube 2B and the first threaded portion 11 of the sheath member 1A are threaded engaged with each other, and then the operation unit 24 is operated to rotate the puncture tube 2B. The blade 22C provided at the distal portion 22B of the puncture tube 2B incises the surface of the application region to perform cutting away of the tissue and takes the tissue into the inner side (FIG. 6B). When the tissue is to be taken out, the inside of the puncture tube 2B is enclosed in a state in which the opening 25 of the puncture tube 2B is closed with a finger or the like, and the puncture tube 2B is pulled out together with the sheath member 1A. By this, the tissue can be exfoliated from the application region.

In this manner, the device according to the third embodiment of the present invention can collect tissue quantitatively by a single time operation without depending upon the manipulation of the operator, and therefore, low invasiveness can be achieved.

Note that the puncture tube 2B is not limited to that of the aforesaid embodiments but can assume various modifications. For example, in addition to the blade at the distal portion 22B of the puncture tube 2B, a spiral blade may be provided in a projecting manner on the inner face of the puncture tube 2B. Also it is possible to make the opening 25 openable and closeable such that the inside of the puncture tube 2B is enclosed without depending upon the manipulation of the operator. Furthermore, the puncture tube 2B may include a piston member (not depicted) that can be inserted into the opening 25. By this, it is possible to slidably move the piston member in the puncture tube 2B to draw up tissue taken in the puncture tube 2B to the proximal portion 23 side or conversely push out the tissue from the distal portion 22 side of the puncture tube 2B.

FIGS. 7A and 7B are sectional views schematically depicting a vessel of the present invention. As depicted in FIG. 7A, a vessel 6 is a bottomed tubular vessel and can accommodate liquid in the inside thereof. The liquid is not restricted, and, for example, liquid medium, physiological saline solution, isotonic solution, buffer solution, cryoprotectant and so forth can be listed. The vessel 6 has an opening 62, and a third threaded portion 61 is provided in a projecting manner on the inner side of the opening 62. The third threaded portion 61 is threaded engageable with the second threaded portion 21 of the puncture tube 2 in the first embodiment, the second threaded portion 41 of the puncture tube 2A in the second embodiment, and the second threaded portion 21 of the puncture tube 2B in the third embodiment. In particular, the vessel 6 can accommodate the puncture tube 2, puncture tube 2A and puncture tube 2B therein. FIG. 7B depicts the vessel 6 in which the puncture tube 2A is accommodated.

By threaded engaging the second threaded portion 41 of the puncture tube 2A with the third threaded portion 61 of the vessel 6, it is possible to threaded engage the puncture tube 2A with certainty in the vessel 6 to place the vessel 6 into an enclosed state. Tissue is taken in the puncture tube 2A and is preserved in a state in which it is immersed in the liquid in the vessel 6. In the case where the state of the tissue reserved in the vessel 6 is to be confirmed, the grasping unit 51 of the inner side tubular body 5 of the puncture tube 2A is gripped and the inner side tubular body 5 is pulled out from the puncture tube 2A. By this, the state of the tissue can be confirmed simply. Here, the vessel and the outer side tubular body 4 may be configured from an optically transparent material such that the state of the tissue can be confirmed from the outside of the vessel.

While the devices according to the embodiments 1 to 3 of the present invention have been described, the present invention is not limited to them. Those skilled in the art can design a device having a different configuration or shape by suitably combining the components and the shapes of the devices according to the aforesaid embodiments 1 to 3. For example, in the present invention, the blade may be provided selectively at the distal of the sheath member 1, at the distal of the puncture tube 2 or at the distal of the plate-shaped member of the puncture tube 2. As the spiral blade, a blade of a spiral shape as is used in an auger screw may be provided in a projecting manner on the inner face of the puncture tube 2 or a spiral blade may be attached to a support like an auger screw so as to be slidably movable in the axial direction of the puncture tube 2.

In the present invention, it is possible to replace the components with arbitrary components that can exhibit similar functions or to add arbitrary components.

### Description of Reference Symbols

- 1, 1A: Sheath member
- 2, 2A, 2B: Puncture tube
- 3: Spiral blade
- 4: Outer side tubular body
- 5: Inner side tubular body
- 6: Vessel

## Claims

1. A tissue collection device comprising:
a sheath member having a first threaded portion on an inner face thereof; and
a puncture tube fitted for back and forth movement in the sheath member and having a second threaded portion provided on an outer face thereof for threaded engaging with the first threaded portion,
wherein the sheath member and/or the puncture tube has a blade at a distal thereof.

2. The tissue collection device according to claim 1, wherein the puncture tube has a spiral blade provided in a projecting manner on an inner face of the puncture tube.

3. The tissue collection device according to claim 1 or 2, wherein the sheath member has graduations on an outer face thereof.

4. The tissue collection device according to any one of claims 1 to 3, wherein the puncture tube includes an outer side tubular body and an inner side tubular body fitted for back and forth movement in the outer side tubular body.

5. The tissue collection device according to claim 4, wherein the inner side tubular body has a groove extending in an axial direction thereof.

6. The tissue collection device according to any one of claims 1 to 5, further comprising:
a vessel for accommodating the puncture tube, the vessel having a third threaded portion for threaded engaging with the second threaded portion.

7. The tissue collection device according to any one of claims 1 to 6, wherein at least part is configured from an optically transparent material.
